(19)

Europäisches Patentamt
European Patent Office
Office européen des brevets

(11) **EP 4 557 174 A1**

(12) **EUROPEAN PATENT APPLICATION**
published in accordance with Art. 153(4) EPC

(43) Date of publication:
**21.05.2025 Bulletin 2025/21**

(21) Application number: **22950599.5**

(22) Date of filing: **14.07.2022**

(51) International Patent Classification (IPC):
*G06N 3/08* (2023.01)

(52) Cooperative Patent Classification (CPC):
**G06N 3/08**

(86) International application number:
**PCT/CN2022/105583**

(87) International publication number:
**WO 2024/011473 (18.01.2024 Gazette 2024/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Boe Technology Group Co., Ltd.**
  **Beijing 100015 (CN)**
- **Beijing BOE Technology Development Co., Ltd.**
  **Beijing 100176 (CN)**

(72) Inventors:
- **LIANG, Shuobin**
  **Beijing 100176 (CN)**

- **CHEN, Yanshun**
  **Beijing 100176 (CN)**
- **LI, Xinguo**
  **Beijing 100176 (CN)**
- **WU, Xinyin**
  **Beijing 100176 (CN)**
- **ZHANG, Honglei**
  **Beijing 100176 (CN)**
- **ZHANG, Xiying**
  **Beijing 100176 (CN)**
- **ZHANG, Zhenzhong**
  **Beijing 100176 (CN)**

(74) Representative: **Potter Clarkson**
**Chapel Quarter**
**Mount Street**
**Nottingham NG1 6HQ (GB)**

(54) **SUBJECT STATE DETERMINATION METHOD, TRAINING METHOD FOR DEEP LEARNING MODEL, AND ELECTRONIC DEVICE**

(57)     A method of determining a state of an object, a method of training a deep learning model, and an electronic device are provided, which relate to a field of an artificial intelligence technology, in particular to a field of a deep learning technology. The method of determining the state of the object includes: performing a feature extraction on a target electroencephalogram signal sequence and a target blood oxygen signal sequence of a target object respectively, so as to obtain target electroencephalogram feature data and target blood oxygen feature data (S210); determining a correlation between the target electroencephalogram feature data and the target blood oxygen feature data (S220); performing a feature fusion on the target electroencephalogram feature data and the target blood oxygen feature data based on the correlation between the target electroencephalogram feature data and the target blood oxygen feature data, so as to obtain target fusion feature data (S230); and determining a current state of the target object according to the target fusion feature data (S240).

200

FIG. 2

**Description**

TECHNICAL FIELD

The present disclosure relates to a field of an artificial intelligence technology, in particular to a field of a deep learning technology. More specifically, the present disclosure relates to a method of determining a state of an object, a method of training a deep learning model, and an electronic device.

BACKGROUND

Brain Computer Interface (BCI) may refer to a direct connection created between a brain of humans or animals and an external device, which is used to achieve an information exchange between the brain and the device. A brain signal detected by a brain computer interface is important data for determining a current state of humans or animals.

SUMMARY

In view of this, the present disclosure provides a method of determining a state of an object, a method of training a deep learning model, an electronic device, a computer readable storage medium, and a computer program product.

According to an aspect of the present disclosure, a method of determining a state of an object is provided, including: performing a feature extraction on a target electroencephalogram signal sequence and a target blood oxygen signal sequence of a target object respectively, so as to obtain target electroencephalogram feature data and target blood oxygen feature data; determining a correlation between the target electroencephalogram feature data and the target blood oxygen feature data; performing a feature fusion on the target electroencephalogram feature data and the target blood oxygen feature data based on the correlation between the target electroencephalogram feature data and the target blood oxygen feature data, so as to obtain target fusion feature data; and determining a current state of the target object according to the target fusion feature data.

According to another aspect of the present disclosure, a method of training a deep learning model is provided, including: performing a feature extraction on a sample electroencephalogram signal sequence and a sample blood oxygen signal sequence of a sample object respectively, so as to obtain sample electroencephalogram feature data and sample blood oxygen feature data; determining a correlation between the sample electroencephalogram feature data and the sample blood oxygen feature data; performing a feature fusion on the sample electroencephalogram feature data and the sample blood oxygen feature data based on the correlation between the sample electroencephalogram feature data and the sample blood oxygen feature data, so as to obtain sample fusion feature data; obtaining a sample state classification result of the sample object according to the sample fusion feature data; and training the deep learning model by using the sample state classification result and a sample state label value, so as to obtain an object state determination model.

According to another aspect of the present disclosure, an electronic device is provided, including: one or more first processors; and a memory for storing one or more programs, wherein the one or more programs are configured to, when executed by the one or more first processors, cause the one or more first processors to implement the methods described in the present disclosure.

According to another aspect of the present disclosure, a computer readable storage medium having computer executable instructions therein is provided, and the instructions are configured to, when executed by a processor, cause the processor to implement the methods described in the present disclosure.

According to another aspect of the present disclosure, a computer program product containing computer executable instructions is provided, wherein the instructions are configured to, when executed, implement the methods described in the present disclosure.

BRIEF DESCRIPTION OF THE DRAWINGS

The above and other objectives, features and advantages of the present disclosure will be clearer with following descriptions of the present disclosure with reference to the accompanying drawings, in which:

FIG. 1 schematically shows a system architecture to which a method of determining a state of an object and a method of training a deep learning model may be applied according to embodiments of the present disclosure;

FIG. 2 schematically shows a flowchart of a method of determining a state of an object according to embodiments of the present disclosure;

FIG. 3A schematically shows an example schematic diagram of displaying a correlation between a target electroencephalogram signal sequence and a target blood oxygen signal sequence by using a predetermined marker according to embodiments of the present disclosure;

FIG. 3B schematically shows an example schematic diagram of displaying the correlation between the target electroencephalogram signal sequence and the target blood oxygen signal sequence in a predetermined form according to embodiments of the present disclosure;

FIG. 4 schematically shows an example schematic diagram of a process of determining a state of an object according to embodiments of the present disclosure;

FIG. 5 schematically shows a flowchart of a method of training a deep learning model according to embodiments of the present disclosure;

FIG. 6 schematically shows an example schematic diagram of a process of training the deep learning model according to embodiments of the present disclosure;

FIG. 7 schematically shows a block diagram of an apparatus of determining a state of an object according to embodiments of the present disclosure;

FIG. 8 schematically shows a block diagram of an apparatus of training a deep learning model according to embodiments of the present disclosure;

FIG. 9A schematically shows a block diagram of an electronic device suitable for implementing the method of determining the state of the object and the method of training the deep learning model according to embodiments of the present disclosure;

FIG. 9B schematically shows a block diagram of an electronic device suitable for implementing the method of determining the state of the object and the method of training the deep learning model according to other embodiments of the present disclosure;

FIG. 9C schematically shows a block diagram of an electronic device suitable for implementing the method of determining the state of the object and the method of training the deep learning model according to other embodiments of the present disclosure;

FIG. 9D schematically shows a block diagram of an electronic device suitable for implementing the method of determining the state of the object and the method of training the deep learning model according to other embodiments of the present disclosure; and

FIG. 10 schematically shows a block diagram of an electronic device suitable for implementing the method of determining the state of the object and the method of training the deep learning model according to other embodiments of the present disclosure.

DETAILED DESCRIPTION OF EMBODIMENTS

Embodiments of the present disclosure will be described below with reference to the accompanying drawings. It should be understood, however, that these descriptions are merely exemplary and are not intended to limit the scope of the present disclosure. In the following detailed description, for ease of interpretation, many specific details are set forth to provide a comprehensive understanding of embodiments of the present disclosure. However, it is clear that one or more embodiments may also be implemented without these specific details. In addition, in the following description, descriptions of well-known structures and technologies are omitted to avoid unnecessarily obscuring the concepts of the present disclosure.

Terms used herein are for the purpose of describing specific embodiments only and are not intended to limit the present disclosure. The terms "including", "containing", etc. used herein indicate the presence of the feature, step, operation and/or component, but do not exclude the presence or addition of one or more other features, steps, operations or components.

All terms used herein (including technical and scientific terms) have the meanings generally understood by those skilled in the art, unless otherwise defined. It should be noted that the terms used herein shall be interpreted to have meanings consistent with the context of this specification, and shall not be interpreted in an idealized or overly rigid manner.

In a case of using the expression similar to "at least one of A, B and C", it should be explained according to the meaning of the expression generally understood by those skilled in the art (for example, "a system including at least one of A, B and C" should include but not be limited to a system including A alone, a system including B alone, a system including C alone, a system including A and B, a system including A and C, a system including B and C, and/or a system including A, B and C). In a case of using the expression similar to "at least one of A, B or C", it should be explained according to the meaning of the expression generally understood by those skilled in the art (for example, "a system including at least one of A, B or C" should include but not be limited to a system including A alone, a system including B alone, a system including C alone, a system including A and B, a system including A and C, a system including B and C, and/or a system including A, B and C).

In technical solutions of the present disclosure, an acquisition, a storage and an application of user personal information involved comply with provisions of relevant laws and regulations, take necessary security measures, and do not violate public order and good custom.

In the technical solutions of the present disclosure, the acquisition or collection of user personal information has been authorized or allowed by users.

A method of detecting a brain signal through a brain computer interface may include an invasive detection and a non-invasive detection. The intrusive detection may include at least one of Ecog or PET (Positron Emission Tomography). The non-invasive detection may include at least one of MEG (Magnetoencephalo-Gram), EEG (Electroencephalo-Gram), fMRI (functional Magnetic Resonance Imaging), or fNIRS (functional Near Infrared Spectrum Instrument), etc.

EEG is achieved based on a volume conduction effect, and electroencephalogram signals received by a plurality of scalp electrodes may come from a same signal source, so that the brain signal detected using EEG may have a low spatial resolution. In addition, due to a slow hemodynamic response of fNIRS after the detection starts, a brain signal detected using fNIRS may have a low temporal resolution.

In summary, a brain signal detection using a single method is difficult to ensure an accuracy of a detection result.

In view of this, the present disclosure provides a solution for determining a state of an object. For example, a feature extraction is performed on a target electroencephalogram signal sequence and a target blood oxygen signal sequence of a target object respectively to obtain target electroencephalogram feature data and target blood oxygen feature data. A correlation between the target electroencephalogram feature data and the target blood oxygen feature data is determined. A feature fusion is performed on the target electroencephalogram feature data and the target blood oxygen feature data based on the correlation between the target electroencephalogram feature data and the target blood oxygen feature data, so as to obtain target fusion feature data. A current state of the target object is determined according to the target fusion feature data.

According to embodiments of the present disclosure, a representation learning ability may be enhanced by performing a feature extraction on the target electroencephalogram signal sequence and the target blood oxygen signal sequence of the target object respectively. On this basis, by obtaining the target fusion feature data through a feature fusion performed based on the correlation between the target electroencephalogram feature data and the target blood oxygen feature data, an accuracy of the target feature fusion data may be improved. In addition, since the current state of the target object is determined according to the obtained target fusion feature data, both the target electroencephalogram feature data and the target blood oxygen feature data of the target object are considered in the determination of the current state of the target object. The target electroencephalogram feature data has a good temporal resolution, and the target blood oxygen feature data has a good spatial resolution, so that the spatial resolution and real-time performance of the signal may be ensured, and the accuracy of the determination of the current state of the target object may be improved.

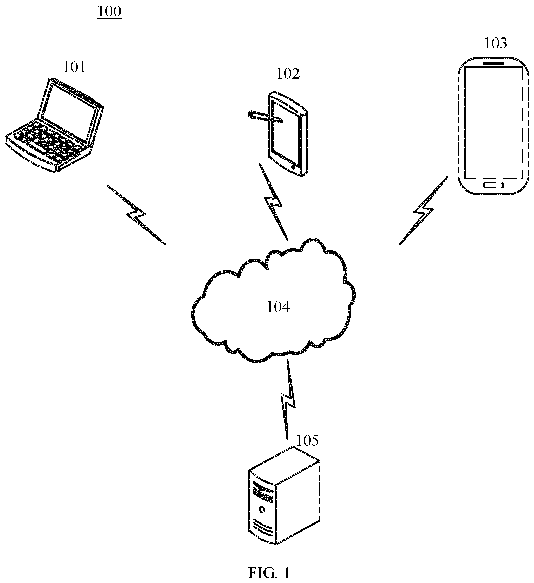

FIG. 1 schematically shows a system architecture to which a method of determining a state of an object and a method of training a deep learning model may be applied according to embodiments of the present disclosure.

It should be noted that FIG. 1 is merely an example of the system architecture to which embodiments of the present disclosure may be applied, so as to help those skilled in the art understand technical contents of the present disclosure. However, it does not mean that embodiments of the present disclosure may not be applied to other devices, systems, environments or scenarios.

As shown in FIG. 1, a system architecture 100 according to such embodiments may include terminal devices 101, 102 and 103, a network 104, and a server 105. The network 104 is a medium for providing a communication link between the terminal devices 101, 102, 103 and the server 105. The network 104 may include various connection types, such as wired and/or wireless communication links, or the like.

The terminal devices 101, 102 and 103 may be used by a user to interact with the server 105 through the network 104 to receive or send messages, etc. The terminal devices 101, 102 and 103 may be installed with various communication client applications, such as shopping applications, web browser applications, search applications, instant messaging tools, email clients and/or social platform software, etc. (just for example).

The terminal devices 101, 102 and 103 may be various electronic devices having display screens and supporting web browsing, including but not limited to smart phones, tablet computers, laptop computers, desktop computers, or the like.

The server 105 may be various types of servers that provide various services. For example, the server 105 may be a cloud server, also known as a cloud computing server or a cloud host, which is a host product in a cloud computing service system to solve shortcomings of difficult management and weak service scalability existing in a conventional physical host and VPS (Virtual Private Server) service. The server 105 may also be a server of a distributed system or a server combined with a block-chain.

It should be noted that the method of determining the state of the object provided in embodiments of the present disclosure may generally be performed by the terminal device 101, 102 or 103. Accordingly, the apparatus of determining the state of the object provided in embodiments of the present disclosure may also be arranged in the terminal device 101, 102 or 103.

Alternatively, the method of determining the state of the object provided in embodiments of the present disclosure may generally be performed by the server 105. Accordingly, the apparatus of determining the state of the object provided in embodiments of the present disclosure may be generally arranged in the server 105. The method of determining the state of the object provided in embodiments of the present disclosure may also be performed by a server or server cluster different from the server 105 and capable of communicating with the terminal devices 101, 102, 103 and/or the server 105. Accordingly, the apparatus of determining the state of the object provided in embodiments of the present disclosure may also be arranged in a server or server cluster different from the server 105 and capable of communicating with the terminal devices 101, 102, 103 and/or the server 105.

It should be noted that the method of training the deep learning model provided by embodiments of the present disclosure may generally be performed by the server 105. Accordingly, the apparatus of training the deep learning model provided in embodiments of the present disclosure may be generally arranged in the server 105. The method of training the deep learning model provided in embodiments of the present disclosure may also be performed by a server or server cluster different from the server 105 and capable of communicating with the terminal devices 101, 102, 103 and/or the server 105. Accordingly, the apparatus of training the deep learning model provided in embodiments of the present disclosure may also be arranged in a server or server cluster different from the server 105 and capable of communicating with the terminal devices 101, 102, 103 and/or the server 105.

Alternatively, the method of training the deep learning model provided in embodiments of the present disclosure may generally be performed by the terminal device 101, 102 or 103. Accordingly, the apparatus of training the deep learning model provided by embodiments of the present disclosure may also be arranged in the terminal device 101, 102 or 103.

It should be understood that the number of terminal devices, network and server shown in FIG. 1 are merely schematic. According to implementation needs, any number of terminal devices, networks and servers may be provided.

It should be noted that a sequence number of each operation in the following methods is merely used to represent the operation for ease of description, and should not be regarded as indicating an execution order of each operation. Unless explicitly stated, the methods do not need to be performed exactly in the order shown.

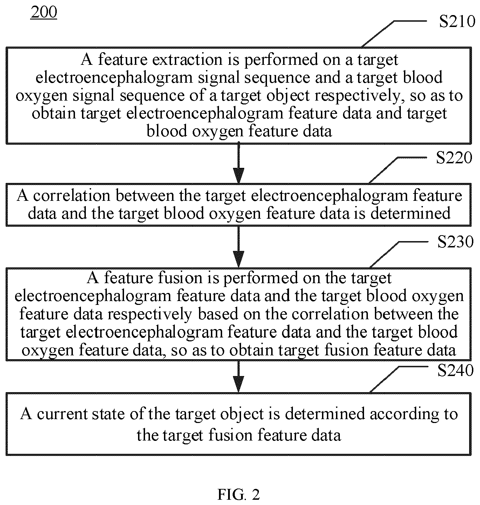

FIG. 2 schematically shows a flowchart of a method of determining a state of an object according to embodiments of the present disclosure.

As shown in FIG. 2, a method 200 of determining a state of an object may include operations S210 to S240.

In operation S210, a feature extraction is performed on a target electroencephalogram signal sequence and a target blood oxygen signal sequence of a target object respectively, so as to obtain target electroencephalogram feature data and target blood oxygen feature data.

In operation S220, a correlation between the target electroencephalogram feature data and the target blood oxygen feature data is determined.

In operation S230, a feature fusion is performed on the target electroencephalogram feature data and the target blood oxygen feature data respectively based on the correlation between the target electroencephalogram feature data and the target blood oxygen feature data, so as to obtain target fusion feature data.

In operation S240, a current state of the target object is determined according to the target fusion feature data.

According to embodiments of the present disclosure, the target object may include humans or animals. An original electroencephalogram signal sequence of the target object may be acquired using an electroencephalogram acquisition device. An original blood oxygen signal sequence of the target object may be acquired using a near-infrared spectrum device.

According to embodiments of the present disclosure, the target electroencephalogram signal sequence may be obtained by pre-processing the original electroencephalogram signal sequence of the target object. The target electroencephalogram signal sequence may include a plurality of target electroencephalogram signals. The target electroencephalogram feature data may be obtained by performing a feature extraction on the target electroencephalogram signal sequence. The target electroencephalogram feature data may include at least one of power spectral density (PSD) feature data, higher-order spectrum (HoS) feature data, or differential entropy (DE) feature data, etc. The target electroencephalogram feature data may include a plurality of target electroencephalogram feature data at a plurality of time instants.

According to embodiments of the present disclosure, the target blood oxygen signal sequence may be obtained by pre-processing the original blood oxygen signal sequence of the target object. The target blood oxygen signal sequence may include a plurality of target blood oxygen signals. The target blood oxygen feature data may be obtained by performing a feature extraction on the target blood oxygen signal sequence. The target blood oxygen feature data may include a plurality of target blood oxygen feature data at a plurality of time instants.

According to embodiments of the present disclosure, the feature extraction performed on the target electroencephalogram signal sequence and the target blood oxygen signal sequence may include at least one of a local feature extraction or a global feature extraction.

According to embodiments of the present disclosure, if a target electroencephalogram signal and a target blood oxygen signal are in a same predetermined time period, it may indicate that the target electroencephalogram feature data corresponding to the target electroencephalogram signal and the target electroencephalogram feature data corresponding to the target blood oxygen signal have a first correlation. If the target electroencephalogram signal and the target blood oxygen signal are not in a same predetermined time period, it may indicate that the target electroencephalogram feature data corresponding to the target electroencephalogram signal and the target blood oxygen feature data corresponding to the target blood oxygen signal have a second correlation. A correlation degree represented by the first correlation is greater than that represented by the second correlation. If the correlation is represented by a weight, the weight corresponding to the first correlation is greater than the weight corresponding to the second correlation. The correlation between the target electroencephalogram feature data and the target blood oxygen feature data may be determined based on an attention strategy, so as to facilitate the feature fusion of the target electroencephalogram feature data and the target blood oxygen feature data based on the correlation between the target electroencephalogram feature data and the target blood oxygen feature data. In addition, the correlation between the target electroencephalogram feature data and the target blood oxygen feature data may also be determined based on other correlation determination strategies. For example, the correlation between the target electroencephalogram feature data and the target blood oxygen feature data may be determined based on a cosine similarity determination strategy; the correlation between the target electroencephalogram feature data and the target blood oxygen feature data may be determined based on a Pearson correlation coefficient strategy; the correlation between the target electroencephalogram feature data and the target blood oxygen feature data may be determined based on a Euclidean distance determination strategy.

According to embodiments of the present disclosure, the current state of the target object may be determined according to the target fusion feature data, which is not limited here. For example, the current state of the target object may include at least one of a sleep state, a motion state, a visual perception state, an auditory perception state, a taste perception state, or a human-machine interaction state, etc. The sleep state may include a sleep quality level.

According to embodiments of the present disclosure, the representation learning ability may be enhanced by performing a feature extraction on the target electroencephalogram signal sequence and the target blood oxygen signal sequence of the target object respectively. On this basis, by obtaining the target fusion feature data through the feature fusion performed based on the correlation between the target electroencephalogram feature data and the target blood oxygen feature data, the accuracy of the target feature fusion data may be improved. In addition, since the current state of the target object is determined according to the obtained target fusion feature data, both the target electroencephalogram feature data and the target blood oxygen feature data of the target object are considered in the determination of the current state of the target object. The target electroencephalogram feature data has a good temporal resolution, and the target blood oxygen feature data has a good spatial resolution, so that the spatial resolution and real-time performance of the signal may be ensured, and the accuracy of the determination of the current state of the target object may be improved.

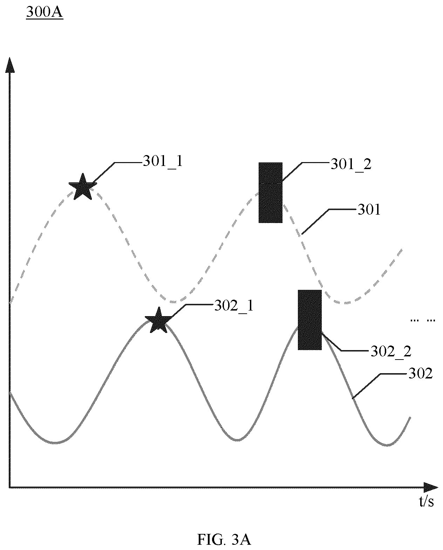

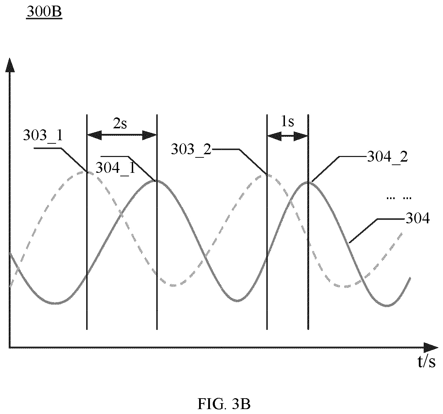

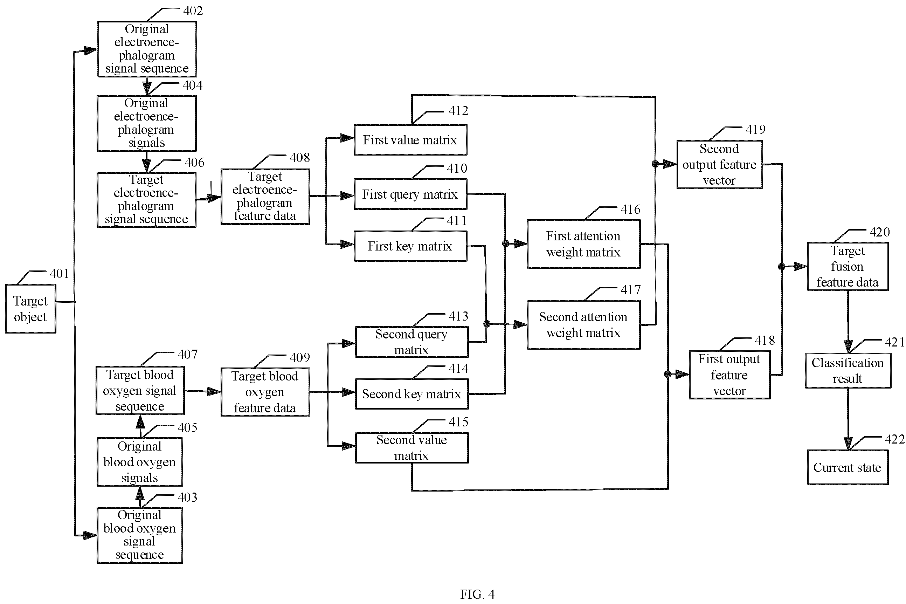

The method of determining the state of the object according to embodiments of the present disclosure will be further described below with reference to FIG. 3A, FIG. 3B and FIG. 4 in conjunction with specific embodiments.

According to embodiments of the present disclosure, the method 200 of determining the state of the object may further include the following operations.

The original electroencephalogram signal sequence and the original blood oxygen signal sequence of the target object are divided respectively to obtain a plurality of original electroencephalogram signals and a plurality of original blood oxygen signals. The target electroencephalogram signal sequence is obtained according to the plurality of original electroencephalogram signals. The target blood oxygen signal sequence is obtained according to the plurality of original blood oxygen signals.

According to embodiments of the present disclosure, after the original electroencephalogram signal sequence and the original blood oxygen signal sequence of the target object are acquired, a pre-processing operation may be performed on the original electroencephalogram signal sequence and the original blood oxygen signal sequence respectively to remove a signal noise and a motion artifact. The pre-processing operation may include at least one of a low-frequency filtering, a high-pass filtering, a baseline correction, or a principal component analysis.

According to embodiments of the present disclosure, a high-frequency signal in the original electroencephalogram signal sequence may be removed through the low-frequency filtering. In addition, due to a low-frequency characteristic of a blood oxygen level dependent (BOLD) response in the original blood oxygen signal sequence, a heartbeat noise and the like in the original blood oxygen signal sequence may be removed through the low-frequency filtering. A signal below a predetermined frequency threshold in the original electroencephalogram signal sequence may be removed through the high-pass filtering, so that a skin potential may be attenuated. For example, the predetermined frequency threshold may include 0.1 Hz.

According to embodiments of the present disclosure, a random division may be performed on the original electroencephalogram signal sequence to obtain a plurality of original electroencephalogram signals, and a random division may be performed on the original blood oxygen signal sequence to obtain a plurality of original blood oxygen signals. Alternatively, the original electroencephalogram signal sequence may be divided based on a sliding window method, so as to obtain a plurality of original electroencephalogram signals. The original blood oxygen signal sequence may be divided based on a sliding window method, so as to obtain a plurality of original blood oxygen signals. The target electroencephalogram signal sequence may include a plurality of original electroencephalogram signals. The target blood oxygen signal sequence may include a plurality of original blood oxygen signals.

According to embodiments of the present disclosure, during the processing of performing the feature extraction on the target electroencephalogram signal sequence and the target blood oxygen signal sequence, the target electroencephalogram feature data corresponding to a current time instant may be fused with the target electroencephalogram feature data at a time instant before the current time instant, that is, the target electroencephalogram feature data at the current time instant carries the target electroencephalogram feature data at the time instant before the current time instant; the target blood oxygen feature data corresponding to the current time instant may be fused with the target blood oxygen feature data at the time instant before the current time instant, that is, the target blood oxygen feature data at the current time instant carries the target blood oxygen feature data at the time instant before the current time instant. Therefore, the original electroencephalogram signal sequence and the original blood oxygen signal sequence are divided to effectively reduce a probability of the correlation between the target electroencephalogram feature data at various time instants and the target blood oxygen feature data at various time instants when subsequently determining the correlation between the target electroencephalogram feature data and the target blood oxygen feature data, so as to ensure the accuracy of the correlation between target electroencephalogram feature data and the target blood oxygen feature data.

According to embodiments of the present disclosure, dividing the original electroencephalogram signal sequence and the original blood oxygen signal sequence of the target object respectively to obtain a plurality of original electroencephalogram signals and a plurality of original blood oxygen signals may include the following operations.

The original electroencephalogram signal sequence and the original blood oxygen signal sequence of the target object are divided respectively based on a sliding window method, so as to obtain the plurality of original electroencephalogram signals and the plurality of original blood oxygen signals. According to embodiments of the present disclosure, a window width and a window level may be set based on the sliding window method, and the original electroencephalogram signal sequence and the original blood oxygen signal sequence of the target object may be divided respectively using different window widths and window levels.

For example, after being aligned, data of the original electroencephalogram signal sequence and data of the original blood oxygen signal sequence may be segmented by a window of a first predetermined duration. Alternatively, after being aligned, the data of the original electroencephalogram signal sequence and the data of the original blood oxygen signal sequence may be segmented by a window of a second predetermined duration and a stride of a third predetermined duration. The third predetermined duration is less than the second predetermined duration. In this way, it may be ensured that a peak of the original electroencephalogram signal and a peak of the original blood oxygen signal are located in a same window, so that more original electroencephalogram signals and original blood oxygen signals may be obtained, and a time interval feature may be retained. The first predetermined duration, the second predetermined duration and the third predetermined duration may be determined according to actual service needs and are not limited here. For example, the first predetermined duration and the second predetermined duration may be 0.4 seconds, and the third predetermined duration may be 0.2 seconds.

According to embodiments of the present disclosure, by dividing the original electroencephalogram signal sequence and the original blood oxygen signal sequence of the target object respectively based on the sliding window method, a plurality of original electroencephalogram signals and a plurality of original blood oxygen signals may be obtained, the accuracy of the correlation between the target electroencephalogram feature data and the target blood oxygen feature data may be ensured, and the accuracy of determining the current state of the target object may be improved.

According to embodiments of the present disclosure, operation S210 may include the following operations.

A local feature extraction is performed on the target electroencephalogram signal sequence and the target blood oxygen signal sequence of the target object respectively, so as to obtain the target electroencephalogram feature data and the target blood oxygen feature data.

According to embodiments of the present disclosure, after the target electroencephalogram signal sequence and the target blood oxygen signal sequence are obtained by dividing the original electroencephalogram signal sequence and the original blood oxygen signal sequence of the target object based on the sliding window method, a local feature extraction may be performed on the target electroencephalogram signal sequence and the target blood oxygen signal sequence of the target object respectively by using a convolutional neural networks (CNN) model, so as to obtain the target electroencephalogram feature data and the target blood oxygen feature data.

According to embodiments of the present disclosure, operation S210 may further include the following operations.

A global feature extraction is performed on the target electroencephalogram signal sequence and the target blood oxygen signal sequence of the target object respectively, so as to obtain the target electroencephalogram feature data and the target blood oxygen feature data.

According to embodiments of the present disclosure, after the target electroencephalogram signal sequence and the target blood oxygen signal sequence are obtained by dividing the original electroencephalogram signal sequence and the original blood oxygen signal sequence of the target object based on the sliding window method, a global feature extraction may be performed on the target electroencephalogram signal sequence and the target blood oxygen signal sequence of the target object respectively by using a recurrent neural network model, so as to obtain the target electroencephalogram feature data and the target blood oxygen feature data. For example, the recurrent neural network model may include a long short-term memory (LSTM) model.

According to embodiments of the present disclosure, operation S210 may further include the following operations.

A local feature extraction is performed on the target electroencephalogram signal sequence and the target blood oxygen signal sequence of the target object respectively, so as to obtain intermediate target electroencephalogram feature data and intermediate target blood oxygen feature data. A global feature extraction is performed on the intermediate target electroencephalogram feature data and the intermediate target blood oxygen feature data respectively, so as to obtain the target electroencephalogram feature data and the target blood oxygen feature data.

According to embodiments of the present disclosure, the local feature extraction and the global feature extraction may be used for a dimension reduction of the target electroencephalogram signal sequence and the target blood oxygen signal sequence.

According to embodiments of the present disclosure, a local feature extraction may be performed on the target electroencephalogram signal sequence and the target blood oxygen signal sequence of the target object respectively by using a convolutional neural network, so as to obtain the intermediate target electroencephalogram feature data and the intermediate target blood oxygen feature data. A global feature extraction may be then performed on the intermediate target electroencephalogram feature data and the intermediate target blood oxygen feature data respectively by using a long short-term memory, so as to obtain the target electroencephalogram feature data and the target blood oxygen feature data.

According to embodiments of the present disclosure, operation S220 may include the following operations.

The correlation between the target electroencephalogram feature data and the target blood oxygen feature data is determine based on an attention strategy.

According to embodiments of the present disclosure, a correlation between two data may be determined based on an attention strategy. For example, an attention layer may be determined based the attention strategy. The target electroencephalogram feature data and the target blood oxygen feature data may be processed using the attention layer, so as to obtain the correlation between the target electroencephalogram feature data and the target blood oxygen feature data.

According to embodiments of the present disclosure, determining the correlation between the target electroencephalogram feature data and the target blood oxygen feature data may include the following operations.

A first query matrix and a first key matrix are determined according to the target electroencephalogram feature data. A second query matrix and a second key matrix are determined according to the target blood oxygen feature data. A first attention weight matrix is determined according to the first query matrix and the second key matrix. A second attention weight matrix is determined according to the second query matrix and the first key matrix. The correlation between the target electroencephalogram feature data and the target blood oxygen feature data is determined according to the first attention weight matrix and the second attention weight matrix.

According to embodiments of the present disclosure, the attention strategy may be represented using Equation (1) shown below.

$$\text{Attention}(Q,\ K,\ V) = \text{softmax} \cdot \left( \frac{QK^{\mathrm{T}}}{\sqrt{d_k}} \right) V \qquad\qquad (1)$$

According to embodiments of the present disclosure, $Q$ may represent a query matrix, $K$ may represent a key matrix, $V$ may represent a value matrix, and $d_k$ may represent a dimensional size of a key matrix.

According to embodiments of the present disclosure, the target electroencephalogram signal sequence may include $N$ target electroencephalogram signals, for example, target electroencephalogram signal $E_1$, target electroencephalogram signal $E_2$,..., target electroencephalogram signal $E_n$,..., target electroencephalogram signal $E_{N-1}$, and target electroencephalogram signal $E_N$. The target blood oxygen signal sequence may include N target blood oxygen signals, for example, target blood oxygen signal $O_1$, target blood oxygen signal $O_2$,..., target blood oxygen signal $O_n$,..., target blood oxygen signal $O_{N-1}$, and target blood oxygen signal $O_N$. $N$ may be an integer greater than 1. $n \in \{1,2,......,N-1, N\}$.

According to embodiments of the present disclosure, after the target electroencephalogram signal sequence is obtained by dividing the original electroencephalogram signal sequence of the target object based on the sliding window method, the target electroencephalogram feature data may be represented by $\{a_1, a_2,......, a_n,......, a_{N-1}, a_N\}$. After the target blood oxygen signal sequence is obtained by dividing the original blood oxygen signal sequence of the target object based on the sliding window method, the target blood oxygen feature data may be represented by $\{b_1, b_2,......, b_n,......, b_{N-1}, b_N\}$. $a_n$ may represent the target electroencephalogram feature data of the target electroencephalogram signal $E_n$. $b_n$ may represent the target blood oxygen feature data of the target blood oxygen signal $O_n$.

According to embodiments of the present disclosure, the target electroencephalogram feature data may be multiplied by $W_1^q$ to obtain a first query matrix $Q_1$. The target electroencephalogram feature data may be multiplied by $W_1^k$ to obtain a first key matrix $K_1$. The target electroencephalogram feature data may be multiplied by $W_1^v$ to obtain a first value matrix $V_1$. The first query matrix $Q_1$ is shown in Equation (2). The first key matrix $K_1$ is shown in Equation (3). The first value matrix $V_1$ is determined by Equation (4). $W_1^q$, $W_1^k$ and $W_1^v$ may represent trainable parameter matrices.

$$Q_1 = q_1^1 q_1^2 \cdots q_1^N = W_1^q \cdot \{a_1, \ a_2,......, \ a_n,......, \ a_{N-1}, \ a_N\} \qquad (2)$$

$$K_1 = k_1^1 k_1^2 \cdots k_1^N = W_1^k \cdot \{a_1, \ a_2,......, \ a_n,......, \ a_{N-1}, \ a_N\} \qquad (3)$$

$$V_1 = v_1^1 v_1^2 \cdots v_1^N = W_1^v \cdot \{a_1, \ a_2,......, \ a_n,......, \ a_{N-1}, \ a_N\} \qquad (4)$$

According to embodiments of the present disclosure, the target blood oxygen feature data may be multiplied by $W_2^q$ to obtain a second query matrix $Q_2$. The target blood oxygen feature data may be multiplied by $W_2^k$ to obtain a second key matrix $K_2$. The target blood oxygen feature data may be multiplied by $W_2^v$ to obtain a second value matrix $V_2$. The second query matrix $Q_2$ is shown in Equation (5). The second key matrix $K_2$ is shown in Equation (6). The second value matrix $V_2$ is determined by Equation (7). $W_2^q$, $W_2^k$ and $W_2^v$ may represent trainable parameter matrices.

$$Q_2 = q_2^1 q_2^2 \cdots q_2^N = W_2^q \cdot \{b_1, \ b_2,......, \ b_n,......, \ b_{N-1}, \ b_N\} \qquad (5)$$

$$K_2 = k_2^1 k_2^2 \cdots k_2^N = W_2^k \cdot \{b_1, \ b_2,......, \ b_n,......, \ b_{N-1}, \ b_N\} \qquad (6)$$

$$V_2 = v_2^1 v_2^2 \cdots v_2^N = W_2^v \cdot \{b_1, \ b_2,......, \ b_n,......, \ b_{N-1}, \ b_N\} \qquad (7)$$

According to embodiments of the present disclosure, a first attention weight matrix $A_1$ may be determined according to the first query matrix $Q_1$ and the second key matrix $K_2$, as shown in Equation (8) below.

$$A_1 = K_2^{\mathrm{T}} \cdot Q_1 \qquad (8)$$

According to embodiments of the present disclosure, a second attention weight matrix $A_2$ may be determined according to the second query matrix $Q_2$ and the first key matrix $K_1$, as shown in Equation (9) below.

$$A_2 = K_1^{\mathrm{T}} \cdot Q_2 \qquad (9)$$

According to embodiments of the present disclosure, operation S230 may include the following operations.

A first output feature vector is obtained according to the first attention weight matrix and the second value matrix, where the second value matrix is obtained according to the target blood oxygen feature data. A second output feature vector is obtained according to the second attention weight matrix and the first value matrix, where the first value matrix is obtained according to the target electroencephalogram feature data. The target fusion feature data is obtained according to the first output feature vector and the second output feature vector.

According to embodiments of the present disclosure, the first output feature vector $C_1$ may be obtained according to the first attention weight matrix $A_1$ and the second value matrix $V_2$, as shown in Equation (10) below.

$$C_1 = \left( v_2^1 \, v_2^2 \cdots v_2^n \right) \cdot A_1^{'} \qquad (10)$$

According to embodiments of the present disclosure, the second output feature vector $C_2$ may be obtained according to the second attention weight matrix $A_2$ and the first value matrix $V_1$, as shown in Equation (11) below.

$$C_2 = \left( v_1^1 \, v_1^2 \cdots v_1^n \right) \cdot A_2^{'} \qquad (11)$$

According to embodiments of the present disclosure, $A_1^{'}$ may be determined according to the first attention weight matrix $A_1$. $A_2^{'}$ may be determined according to the second attention weight matrix $A_2$. For example, $A_1$ may be processed using an activation function to obtain $A_1^{'}$. $A_2$ may be processed using an activation function to obtain $A_2^{'}$.

According to embodiments of the present disclosure, the first output feature vector $C_1$ and the second output feature vector $C_2$ may be concatenated according to the first output feature vector $C_1$ and the second output feature vector $C_2$, so as to obtain the target fusion feature data $c$, as shown in Equation (12) below.

$$c = contact(C_1, C_2) \qquad (12)$$

According to embodiments of the present disclosure, by determining the correlation between the target electroencephalogram feature data and the target blood oxygen feature data based on the attention strategy, the feature fusion may be performed on the target electroencephalogram feature data and the target blood oxygen feature data based on the correlation, so that the accuracy of the target feature fusion data may be improved.

According to embodiments of the present disclosure, the method 200 of determining the state of the object may further include the following operations.

The correlation between the target electroencephalogram signal sequence and the target blood oxygen signal sequence is displayed using a predetermined marker. The correlation between the target electroencephalogram signal sequence and the target blood oxygen signal sequence is determined according to the target electroencephalogram feature data and the target blood oxygen feature data.

According to embodiments of the present disclosure, the correlation between the target electroencephalogram signal sequence and the target blood oxygen signal sequence may be determined according to the target electroencephalogram feature data corresponding to the target electroencephalogram signal sequence and the target blood oxygen feature data corresponding to the target blood oxygen signal sequence, that is, the correlation between the target electroencephalogram feature data corresponding to the target electroencephalogram signal sequence and the target blood oxygen feature data corresponding to the target blood oxygen signal sequence may be used to represent the correlation between the target electroencephalogram signal sequence and the target blood oxygen signal sequence.

According to embodiments of the present disclosure, the target electroencephalogram signal sequence may include a plurality of target electroencephalogram signals, and the target blood oxygen signal sequence may include a plurality of target blood oxygen signals. For each of the plurality of target electroencephalogram signals, the target electroencephalogram signal and a target blood oxygen signal that is correlated with the target electroencephalogram signal may be displayed using a predetermined marker. The target electroencephalogram signals may correspond to different predetermined markers. The predetermined marker may include at least one of a regular shape marker or an irregular shape marker. The regular shape marker may include at least one of a rectangular marker, a star marker, a circular marker, a triangle marker, an elliptical marker, or a rhombic marker. A color and a line-type of the predetermined marker may be determined according to actual service needs and are not limited here.

According to embodiments of the present disclosure, by displaying the correlation between the target electroencephalogram signal sequence and the target blood oxygen signal sequence using the predetermined marker, the correlation between the target electroencephalogram signal sequence and the target blood oxygen signal sequence may be intuitively obtained.

According to embodiments of the present disclosure, the method 200 of determining the state of the object may further include the following operations.

The target electroencephalogram signal sequence and the target blood oxygen signal sequence are displayed.

According to embodiments of the present disclosure, the target electroencephalogram signal sequence and the target blood oxygen signal sequence may be displayed in a predetermined form. The predetermined form may be determined according to actual service needs and is not limited here. For example, the predetermined form may include a superimposition form. Alternatively, the predetermined form may include a superimposition form in which a time difference between a peak corresponding to the target electroencephalogram signal and a peak corresponding to the target blood oxygen signal correlated with the target electroencephalogram signal is displayed.

FIG. 3A schematically shows an example schematic diagram of displaying the correlation between the target electroencephalogram signal sequence and the target blood oxygen signal sequence by using a predetermined marker according to embodiments of the present disclosure.

As shown in FIG. 3A, in 300A, a target electroencephalogram signal sequence 301 may include a target electroencephalogram signal 301_1, a target electroencephalogram signal 301_2, ... A target blood oxygen signal sequence 302 may include a target blood oxygen signal 302_1, a target blood oxygen signal 302_2, ... The target electroencephalogram signal 301_1 and the target blood oxygen signal 302_1 have a correlation, and the target electroencephalogram signal 301_2 and the target blood oxygen signal 302_2 have a correlation.

The correlation between the target electroencephalogram signal 301_1 and the target blood oxygen signal 302_1 may be displayed using a "star marker". The correlation between the target electroencephalogram signal 301_2 and the target blood oxygen signal 302_2 may be displayed using a "rectangular marker".

FIG. 3B schematically shows an example schematic diagram of displaying the correlation between the target electroencephalogram signal sequence and the target blood oxygen signal sequence in a predetermined form according to embodiments of the present disclosure. As shown in FIG. 3B, in 300B, a target electroencephalogram signal sequence 303 and a target blood oxygen signal sequence 304 may be displayed in a superimposition form. The target electroencephalogram signal sequence 303 may include a target electroencephalogram signal 303_1, a target electroencephalogram signal 303_2, ... The target blood oxygen signal sequence 304 may include a target blood oxygen signal 304_1, a target blood oxygen signal 304_2, ... The target electroencephalogram signal 303_1 and the target blood oxygen signal 304_1 have a correlation, and the target electroencephalogram signal 303_2 and the target blood oxygen signal 304_2 have a correlation.

The time difference between a peak corresponding to the target electroencephalogram signal 303_1 and a peak corresponding to the target blood oxygen signal 304_1 is 2 seconds. The time difference between a peak corresponding to the target electroencephalogram signal 303_2 and a peak corresponding to the target blood oxygen signal 304_2 is 1 second.

According to embodiments of the present disclosure, operation S240 may include the following operations.

A classification result is obtained according to the target fusion feature data. The current state of the target object is determined according to the classification result.

According to embodiments of the present disclosure, after the target fusion feature data is obtained, the target fusion feature data may be input into a deep learning model to output the classification result. The deep learning model may include at least one of a support vector machine, a random forest, or a decision tree, etc.

FIG. 4 schematically shows an example schematic diagram of a process of determining a state of an object according to embodiments of the present disclosure.

As shown in FIG. 4, an original electroencephalogram signal sequence 402 and an original blood oxygen signal sequence 403 of a target object 401 may be divided respectively based on a sliding window method, so as to obtain a plurality of original electroencephalogram signals 404 and a plurality of original blood oxygen signals 405. A target electroencephalogram signal sequence 406 is obtained according to the plurality of original electroencephalogram signals 404. A target blood oxygen signal sequence 407 is obtained according to the plurality of original blood oxygen signals 405.

A feature extraction may be performed on the target electroencephalogram signal sequence 406 of the target object 401 to obtain target electroencephalogram feature data 408, and a feature extraction may be performed on the target blood oxygen signal sequence 407 of the target object 401 to obtain target blood oxygen feature data 409.

A first query matrix 410 and a first key matrix 411 may be determined according to the target electroencephalogram feature data 408. A second query matrix 413 and a second key matrix 414 may be determined according to the target blood oxygen feature data 409. A first attention weight matrix 416 may be determined according to the first query matrix 410 and the second key matrix 414. A second attention weight matrix 417 may be determined according to the second query matrix 413 and the first key matrix 411. A correlation between the target electroencephalogram feature data 408 and the target blood oxygen feature data 409 may be determined according to the first attention weight matrix 416 and the second attention weight matrix 417.

A first output feature vector 418 may be obtained according to the first attention weight matrix 416 and the second value matrix 415 that is obtained from the target blood oxygen feature data 409. A second output feature vector 419 may be obtained according to the second attention weight matrix 417 and the first value matrix 412 that is obtained from the target electroencephalogram feature data 408. Target fusion feature data 420 may be obtained according to the first output feature vector 418 and the second output feature vector 419.

A classification result 421 may be obtained according to the target fusion feature data 420. A current state 422 of the target object 401 may be determined according to the classification result 421.

The above are merely exemplary embodiments. The present disclosure is not limited thereto, and may further include other methods of determining a state of an object known in the art, as long as the state of the object may be determined.

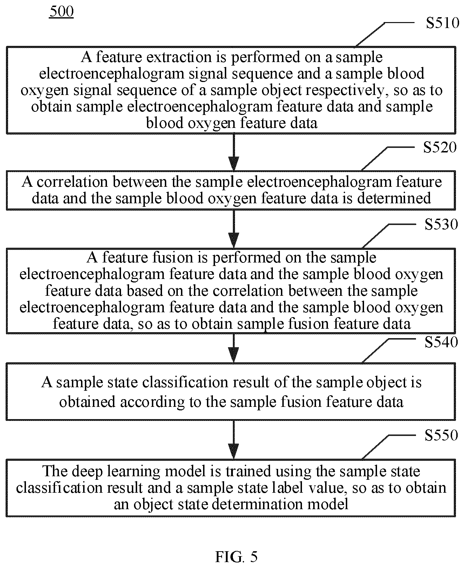

FIG. 5 schematically shows a flowchart of a method of training a deep learning model according to embodiments of the present disclosure.

As shown in FIG. 5, a method 500 of training a deep learning model may include operations S510 to S550.

In operation S510, a feature extraction is performed on a sample electroencephalogram signal sequence and a sample blood oxygen signal sequence of a sample object respectively, so as to obtain sample electroencephalogram feature data and sample blood oxygen feature data.

In operation S520, a correlation between the sample electroencephalogram feature data and the sample blood oxygen feature data is determined.

In operation S530, a feature fusion is performed on the sample electroencephalogram feature data and the sample blood oxygen feature data based on the correlation between the sample electroencephalogram feature data and the sample blood oxygen feature data, so as to obtain sample fusion feature data.

In operation S540, a sample state classification result of the sample object is obtained according to the sample fusion feature data.

In operation S550, the deep learning model is trained using the sample state classification result and a sample state label value, so as to obtain an object state determination model.

According to embodiments of the present disclosure, the sample object may include humans, the original sample electroencephalogram signal sequence of the sample object may be obtained using an electroencephalogram acquisition device, and the original sample blood oxygen signal sequence of the sample object may be obtained using a near-infrared spectrum device.

According to embodiments of the present disclosure, the deep learning model may include a feature extraction module and a classification module. Model structures of the feature extraction module and the classification module may be determined according to actual service needs and are not limited here. For example, the feature extraction module may include at least one of: a feature extraction module based on a convolutional neural network model, or a feature extraction module based on a recurrent neural network model. The recurrent neural network model may include a long short-term memory model. The classification module may include a support vector machine, a random forest, or a decision tree.

According to embodiments of the present disclosure, the sample electroencephalogram signal sequence may be obtained by pre-processing the original sample electroencephalogram signal sequence of the sample object, and the sample electroencephalogram feature data may be obtained by performing a feature extraction on the sample electroencephalogram signal sequence.

According to embodiments of the present disclosure, the sample blood oxygen signal sequence may be obtained by pre-processing the original sample blood oxygen signal sequence of the sample object, and the sample blood oxygen feature data may be obtained by performing a feature extraction on the sample blood oxygen signal sequence.

According to embodiments of the present disclosure, the feature extraction performed on the sample electroencephalogram signal sequence and the sample blood oxygen signal sequence may include at least one of a local feature extraction or a global feature extraction.

According to embodiments of the present disclosure, the correlation between the sample electroencephalogram feature data and the sample blood oxygen feature data may be determined based on an attention mechanism, so that a feature fusion may be performed on the sample electroencephalogram feature data and the sample blood oxygen feature data based on the correlation between the sample electroencephalogram feature data and the sample blood oxygen feature data.

According to embodiments of the present disclosure, a sample state classification result of the sample object may be determined according to the sample fusion feature data and is not limited here. For example, the sample state classification result of the sample object may include at least one of a sleep state, a motion state, a visual perception state, an auditory perception state, a taste perception state, or a human-machine interaction state. The sleep state may include a sleep quality level.

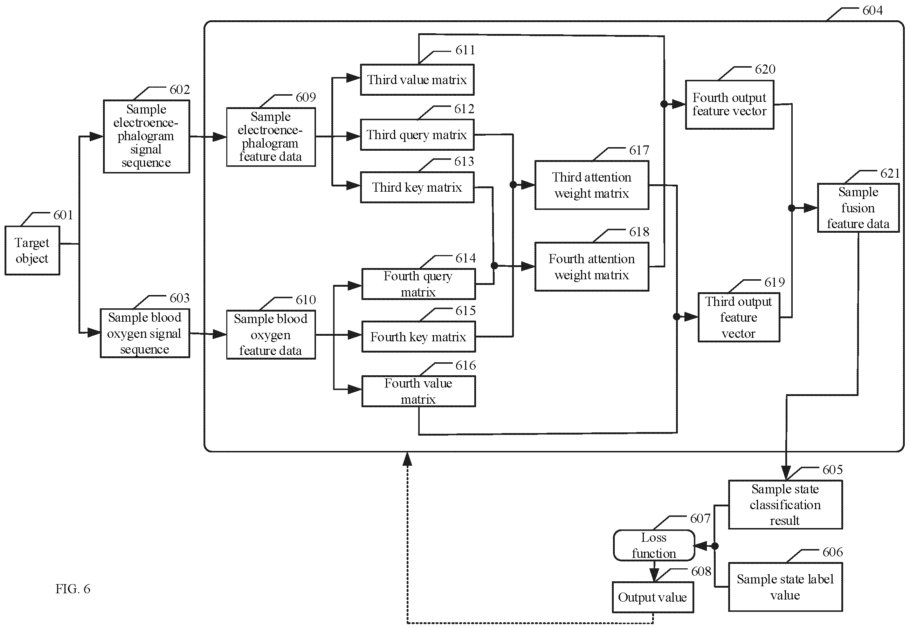

The method of training the deep learning model according to embodiments of the present disclosure will be further described below with reference to FIG. 6 in conjunction with specific embodiments.

According to embodiments of the present disclosure, the deep learning model may include a first local feature extraction module.

According to embodiments of the present disclosure, operation S510 may include the following operations.

The sample electroencephalogram signal sequence and the sample blood oxygen signal sequence of the sample object are processed respectively using the first local feature extraction module, so as to obtain the sample electroencephalogram feature data and the sample blood oxygen feature data.

According to embodiments of the present disclosure, the first global feature extraction module may be, for example, a convolutional neural network. After the sample electroencephalogram signal sequence and the sample blood oxygen signal sequence are acquired, a local feature extraction may be performed on the sample electroencephalogram signal sequence and the sample blood oxygen signal sequence of the sample object respectively by using a convolutional neural network, so as to obtain the sample electroencephalogram feature data and the sample blood oxygen feature data.

According to embodiments of the present disclosure, the deep learning model may include a first global feature extraction module.

According to embodiments of the present disclosure, operation S510 may include the following operations.

The sample electroencephalogram signal sequence and the sample blood oxygen signal sequence of the sample object are processed respectively using the first global feature extraction module, so as to obtain the sample electroencephalogram feature data and the sample blood oxygen feature data.

According to embodiments of the present disclosure, the first global feature extraction module may be, for example, a long short-term memory. After the sample electroencephalogram signal sequence and the sample blood oxygen signal sequence are acquired, a global feature extraction may be performed on the sample electroencephalogram signal sequence and the sample blood oxygen signal sequence of the sample object by using a long short-term memory, so as to obtain the sample electroencephalogram feature data and the sample blood oxygen feature data.

According to embodiments of the present disclosure, the deep learning model may include a second local feature extraction module and a second global feature extraction module.

According to embodiments of the present disclosure, operation S510 may include the following operations.

The sample electroencephalogram signal sequence and the sample blood oxygen signal sequence of the sample object are processed respectively using the second local feature extraction module, so as to obtain intermediate sample electroencephalogram feature data and intermediate sample blood oxygen feature data. The intermediate sample electroencephalogram feature data and the intermediate sample blood oxygen feature data are processed respectively using the second global feature extraction module, so as to obtain the sample electroencephalogram feature data and the sample blood oxygen feature data.

According to embodiments of the present disclosure, the second global feature extraction module may be, for example, a convolutional neural network. The second global feature extraction module may be, for example, a long short-term memory. The local feature extraction and the global feature extraction may be used for a dimension reduction of the sample electroencephalogram signal sequence and the sample blood oxygen signal sequence.

According to embodiments of the present disclosure, a local feature extraction may be performed on the sample electroencephalogram signal sequence and the sample blood oxygen signal sequence of the sample object respectively by using a convolutional neural network, so as to obtain the intermediate sample electroencephalogram feature data and the intermediate sample blood oxygen feature data. Then, a global feature extraction may be performed on the intermediate sample electroencephalogram feature data and the intermediate sample blood oxygen feature data respectively by using a long short-term memory, so as to obtain the sample electroencephalogram feature data and the sample blood oxygen feature data.

According to embodiments of the present disclosure, the deep learning model may include an attention module.

According to embodiments of the present disclosure, operation S520 may include the following operations.

The sample electroencephalogram feature data and the sample blood oxygen feature data are processed using the attention module, so as to obtain the correlation between the sample electroencephalogram feature data and the sample blood oxygen feature data.

According to embodiments of the present disclosure, processing the sample electroencephalogram feature data and the sample blood oxygen feature data using the attention module to obtain the correlation between the sample electroencephalogram feature data and the sample blood oxygen feature data may include the following operations.

A third query matrix and a third key matrix are determined according to the sample electroencephalogram feature data. A fourth query matrix and a fourth key matrix are determined according to the sample blood oxygen feature data. A third attention weight matrix is determined according to the third query matrix and the fourth key matrix. A fourth attention weight matrix is determined according to the fourth query matrix and the third key matrix. The correlation between the sample electroencephalogram feature data and the sample blood oxygen feature data is determined according to the third attention weight matrix and the fourth attention weight matrix.

According to embodiments of the present disclosure, operation S530 may include the following operations.

A third output feature vector is obtained according to the third attention weight matrix and a fourth value matrix, where the fourth value matrix is obtained according to the sample blood oxygen feature data. A fourth output feature vector is obtained according to the fourth attention weight matrix and a third value matrix, where the third value matrix is obtained according to the sample electroencephalogram feature data. The sample fusion feature data is obtained according to the third output feature vector and the fourth output feature vector.

According to embodiments of the present disclosure, by determining the correlation between the sample electroencephalogram feature data and the sample blood oxygen feature data based on the attention strategy, the feature fusion may be performed on the sample electroencephalogram feature data and the sample blood oxygen feature data based on the correlation, so that the accuracy of the sample fusion feature data may be improved.

According to embodiments of the present disclosure, operation S550 may include the following operations.

The sample state classification result and the sample state label value are input into a loss function to obtain an output value. A model parameter of the deep learning model is adjusted according to the output value until a predetermined end condition is met, so as to obtain the object state determination model.

According to embodiments of the present disclosure, the output value may be obtained based on the loss function by using the sample state classification result and the sample state label value. The model parameter of the deep learning model may be adjusted according to the output value until a predetermined condition is met. For example, the model parameter of the deep learning model may be adjusted according to a back-propagation algorithm or a random gradient descent algorithm until the predetermined end condition is met. The deep learning model obtained when the predetermined end condition is met may be determined as the object state determination model.

According to embodiments of the present disclosure, the loss function may include, for example, a Hinge loss function, an exponential loss function, a square loss function, or a cross entropy loss function. The predetermined end condition may include at least one of a convergence of the output value or a maximum number of training rounds being reached.

According to embodiments of the present disclosure, the method of training the deep learning model may further include repeatedly performing the following operations until a performance test result of the object state determination model meets a predetermined performance condition.

A model performance of the object state determination model is tested using a validation signal sequence to obtain the performance test result. A model hyper-parameter corresponding to the object state determination model is adjusted when it is determined that the performance test result does not meet the predetermined performance condition. The deep learning model is re-trained based on the adjusted model hyper-parameter by using the sample electroencephalogram signal sequence, the sample blood oxygen signal sequence and the sample state label value, so as to obtain a new object state determination model.

According to embodiments of the present disclosure, the model performance may be represented by a model performance evaluation value. The performance test result includes the model performance evaluation value. The model performance evaluation value may include at least one of precision, recall, accuracy, error rate, or F-function value. The predetermined performance condition may refer to that the performance evaluation value is greater than or equal to a predetermined performance evaluation threshold. The predetermined performance evaluation threshold may be determined according to actual service needs and is not limited here. The model hyper-parameter may include at least one of a learning rate or a number of layers of the deep learning model, etc.

According to embodiments of the present disclosure, the model performance of the object state determination model is tested using a validation signal sequence to obtain the performance test result. For example, the validation signal sequence may be processed using the object state determination model, so as to obtain a state classification result. The performance test result may be determined according to the state classification result. It is determined whether the performance test result meets the predetermined performance condition or not. When it is determined that the performance test result meets the predetermined performance condition, a training operation of the deep learning model ends. When it is determined that the performance test result does not meet the predetermined performance condition, the model hyper-parameter corresponding to the object state determination model may be adjusted. The deep learning model may be re-trained based on the adjusted model hyper-parameter by using the sample electroencephalogram signal sequence, the sample blood oxygen signal sequence and the sample state label value, so as to obtain a new object state determination model. The above operations may be repeatedly performed until the performance test result meets the predetermined performance condition.

FIG. 6 schematically shows an example schematic diagram of a process of training the deep learning model according to embodiments of the present disclosure.

As shown in FIG. 6, a feature extraction may be performed on a sample electroencephalogram signal sequence 602 of a sample object 601 to obtain sample electroencephalogram feature data 609. A feature extraction may be performed on a sample blood oxygen signal sequence 603 of the sample object 601 to obtain sample blood oxygen feature data 610.

A third query matrix 612 and a third key matrix 613 may be determined according to the sample electroencephalogram feature data 609. A fourth query matrix 614 and a fourth key matrix 615 may be determined according to the sample blood oxygen feature data 610. A third attention weight matrix 617 may be determined according to the third query matrix 612 and the fourth key matrix 615. A fourth attention weight matrix 618 may be determined according to the fourth query matrix 614 and the third key matrix 613. A correlation between the sample electroencephalogram feature data and the sample blood oxygen feature data may be determined according to the third attention weight matrix 617 and the fourth attention weight matrix 618.

A third output feature vector 619 may be obtained according to the third attention weight matrix 617 and the fourth value matrix 616 that is obtained from the sample blood oxygen feature data 609. A fourth output feature vector 620 may be obtained according to the fourth attention weight matrix 618 and the third value matrix 611 that is obtained from the sample electroencephalogram feature data 610. Sample fusion feature data 621 may be obtained according to the third output feature vector 619 and the fourth output feature vector 620.

A sample state classification result 605 of the sample object 601 may be obtained according to the sample fusion feature data 621. The sample state classification result 605 and a sample state label value 606 may be input into a loss function 607 to obtain an output value 608. A model parameter of the deep learning model 604 may be adjusted according to the output value 608 until a predetermined end condition is met, so as to obtain an object state determination model.

The above are merely exemplary embodiments. The present disclosure is not limited thereto, and may further include other methods of training a deep learning model known in the art, as long as the deep learning model may be trained.

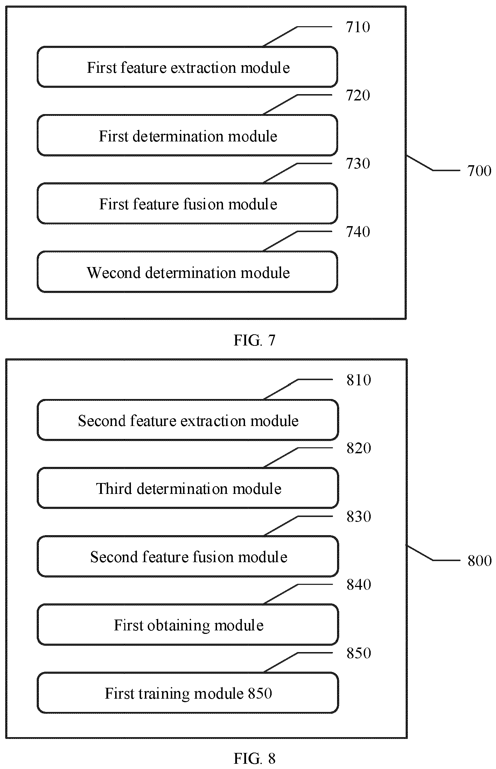

FIG. 7 schematically shows a block diagram of an apparatus of determining a state of an object according to embodiments of the present disclosure.

As shown in FIG. 7, an apparatus 700 of determining a state of an object may include a first feature extraction module 710, a first determination module 720, a first feature fusion module 730, and a second determination module 740.

The first feature extraction module 710 is used to perform a feature extraction on a target electroencephalogram signal sequence and a target blood oxygen signal sequence of a target object respectively, so as to obtain target electroencephalogram feature data and target blood oxygen feature data.

The first determination module 720 is used to determine a correlation between the target electroencephalogram feature data and the target blood oxygen feature data.

The first feature fusion module 730 is used to perform a feature fusion on the target electroencephalogram feature data and the target blood oxygen feature data based on the correlation between the target electroencephalogram feature data and the target blood oxygen feature data, so as to obtain target fusion feature data.

The second determination module 740 is used to determine a current state of the target object according to the target fusion feature data.

According to embodiments of the present disclosure, the first determination module 720 may include a first determination unit, a second determination unit, a third determination unit, a fourth determination unit, and a fifth determination unit.

The first determination is used to determine a first query matrix and a first key matrix according to the target electroencephalogram feature data.

The second determination is used to determine a second query matrix and a second key matrix according to the target blood oxygen feature data.

The third determination is used to determine a first attention weight matrix according to the first query matrix and the second key matrix.

The fourth determination is used to determine a second attention weight matrix according to the second query matrix and the first key matrix.

The fifth determination is used to determine the correlation between the target electroencephalogram feature data and the target blood oxygen feature data according to the first attention weight matrix and the second attention weight matrix.

According to embodiments of the present disclosure, the first feature fusion module 730 may include a first obtaining unit, a second obtaining unit, and a third obtaining unit.

The first obtaining unit is used to obtain a first output feature vector according to the first attention weight matrix and a second value matrix. The second value matrix is obtained according to the target blood oxygen feature data.

The second obtaining unit is used to obtain a second output feature vector according to the second attention weight matrix and a first value matrix. The first value matrix is obtained according to the target electroencephalogram feature data.

The third obtaining unit is used to obtain the target fusion feature data according to the first output feature vector and the second output feature vector.

According to embodiments of the present disclosure, the apparatus 700 of determining the state of the object may further include a dividing module, a second obtaining module, and a third obtaining module.

The dividing module is used to divide an original electroencephalogram signal sequence and an original blood oxygen signal sequence of the target object respectively, so as to obtain a plurality of original electroencephalogram signals and a plurality of original blood oxygen signals.

The second obtaining module is used to obtain the target electroencephalogram signal sequence according to the plurality of original electroencephalogram signals.

The third obtaining module is used to obtain the target blood oxygen signal sequence according to the plurality of original blood oxygen signals.

According to embodiments of the present disclosure, the dividing module may include a dividing unit.

The dividing unit is used to divide the original electroencephalogram signal sequence and the original blood oxygen signal sequence of the target object respectively based on a sliding window method, so as to obtain the plurality of original electroencephalogram signals and the plurality of original blood oxygen signals.

According to embodiments of the present disclosure, the first feature extraction module 710 may include a first local feature extraction unit.

The first local feature extraction unit is used to perform a local feature extraction on the target electroencephalogram signal sequence and the target blood oxygen signal sequence of the target object respectively, so as to obtain the target electroencephalogram feature data and the target blood oxygen feature data.

According to embodiments of the present disclosure, the first feature extraction module 710 may include a first global feature extraction unit.

The first global feature extraction unit is used to perform a global feature extraction on the target electroencephalogram signal sequence and the target blood oxygen signal sequence of the target object respectively, so as to obtain the target electroencephalogram feature data and the target blood oxygen feature data.

According to embodiments of the present disclosure, the first feature extraction module 710 may include a second local feature extraction unit and a second global feature extraction unit.

The second local feature extraction unit is used to perform a local feature extraction on the target electroencephalogram signal sequence and the target blood oxygen signal sequence of the target object respectively, so as to obtain intermediate target electroencephalogram feature data and intermediate target blood oxygen feature data.

The second global feature extraction unit is used to perform a global feature extraction on the intermediate target electroencephalogram feature data and the intermediate target blood oxygen feature data respectively, so as to obtain the target electroencephalogram feature data and the target blood oxygen feature data.

According to embodiments of the present disclosure, the second determination module 740 may include a sixth determination unit.

The sixth determination unit is used to obtain a classification result according to the target fusion feature data; and determine the current state of the target object according to the classification result.

According to embodiments of the present disclosure, the apparatus 700 of determining the state of the object may further include a display module.

The display module is used to display the correlation between the target electroencephalogram signal sequence and the target blood oxygen signal sequence by using a predetermined marker. The correlation between the target electroencephalogram signal sequence and the target blood oxygen signal sequence is determined according to the target electroencephalogram feature data and the target blood oxygen feature data.

FIG. 8 schematically shows a block diagram of an apparatus of training a deep learning model according to embodiments of the present disclosure.

As shown in FIG. 8, an apparatus 700 of training a deep learning model may include a second feature extraction module 810, a third determination module 820, a second feature fusion module 830, a first obtaining module 840, and a first training module 850.

The second feature extraction module 810 is used to perform a feature extraction on a sample electroencephalogram signal sequence and a sample blood oxygen signal sequence of a sample object respectively, so as to obtain sample electroencephalogram feature data and sample blood oxygen feature data.

The third determination module 820 is used to determine a correlation between the sample electroencephalogram feature data and the sample blood oxygen feature data.

The second feature fusion module 830 is used to perform a feature fusion on the sample electroencephalogram feature data and the sample blood oxygen feature data based on the correlation between the sample electroencephalogram feature data and the sample blood oxygen feature data, so as to obtain sample fusion feature data.

The first obtaining module 840 is used to obtain a sample state classification result of the sample object according to the sample fusion feature data.

The first training module 850 is used to train the deep learning model by using the sample state classification result and a sample state label value, so as to obtain an object state determination model.

According to embodiments of the present disclosure, the first training module 850 may include an input unit and an adjustment unit.

The input unit is used to input the sample state classification result and the sample state label value into a loss function to obtain an output value.

The adjustment unit is used to adjust a model parameter of the deep learning model according to the output value until a predetermined end condition is met, so as to obtain the object state determination model.

According to embodiments of the present disclosure, the apparatus 700 of training the deep learning model may further include a testing module, an adjustment module, and a second training module, which are used to repeatedly perform the following operations until a performance test result of the object state determination model meets a predetermined performance condition.

The testing module is used to test a model performance of the object state determination model by using a validation signal sequence, so as to obtain the performance test result.

The adjustment module is used to adjust a model hyper-parameter corresponding to the object state determination model to obtain an adjusted model hyper-parameter, in response to a determination that the performance test result does not meet the predetermined performance condition.

The second training module is used to re-train the deep learning model based on the adjusted model hyper-parameter by using the sample electroencephalogram signal sequence, the sample blood oxygen signal sequence and the sample state label value, so as to obtain a new object state determination model.

Any number of the modules, sub-modules and units according to embodiments of the present disclosure, or at least part of functions of any number of them may be implemented in one module. Any one or more of the modules, sub-modules and units according to embodiments of the present disclosure may be split into a plurality of modules for implementation. Any one or more of the modules, sub-modules and units according to embodiments of the present disclosure may be implemented at least partially as a hardware circuit, such as a field programmable gate array (FPGA), a programmable logic array (PLA), a system on a chip, a system on a substrate, a system on a package, an Application Specific Integrated Circuit (ASIC), or may be implemented by hardware or firmware in any other reasonable manner of integrating or encapsulating the circuit, or may be implemented by any one of three implementation modes of software, hardware and firmware or an appropriate combination thereof. Alternatively, one or more of the modules, sub-modules and units according to embodiments of the present disclosure may be at least partially implemented as a computer program module that, when executed, performs the corresponding functions.

For example, any number of the first feature extraction module 710, the first determination module 720, the first feature fusion module 730 and the second determination module 740, or any number of the second feature extraction module 810, the third determination module 820, the second feature fusion module 830, the first obtaining module 840 and the first training module 850 may be combined into one module/unit for implementation, or any one of the modules/units may be divided into a plurality of modules/units. Alternatively, at least part of the functions of one or more of these modules/units may be combined with at least part of the functions of other modules/sub-modules/units and implemented in one module/sub-module/unit. According to embodiments of the present disclosure, at least one of the first feature extraction module 710, the first determination module 720, the first feature fusion module 730 and the second determination module 740, or at least one of the second feature extraction module 810, the third determination module 820, the second feature fusion module 830, the first obtaining module 840 and the first training module 850 may be implemented at least partially as a hardware circuit, such as a field programmable gate array (FPGA), a programmable logic array (PLA), a system on a chip, a system on a substrate, a system on a package, an Application Specific Integrated Circuit (ASIC), or may be implemented by hardware or firmware in any other reasonable manner of integrating or encapsulating the circuit, or may be implemented by any one of the three implementation modes of software, hardware and firmware or an appropriate combination thereof. Alternatively, at least one of the first feature extraction module 710, the first determination module 720, the first feature fusion module 730 and the second determination module 740, or at least one of the second feature extraction module 810, the third determination module 820, the second feature fusion module 830, the first obtaining module 840 and the first training module 850 may be at least partially implemented as a computer program module that may perform corresponding functions when executed.

It should be noted that a part for the apparatus of determining the state of the object in embodiments of the present disclosure corresponds to a part for the method of determining the state of the object in embodiments of the present disclosure. For the descriptions of the apparatus of determining the state of the object, reference may be made to the method of determining the state of the object, and details will not be repeated here. A part for the apparatus of training the deep learning model in embodiments of the present disclosure corresponds to a part for the method of training the deep learning model in embodiments of the present disclosure. For the descriptions of the apparatus of training the deep learning model, reference may be made to the method of training the deep learning model, and details will not be repeated here.

According to embodiments of the present disclosure, the electronic device may further include a signal acquisition module.

The signal acquisition module includes at least one signal acquisition unit. The signal acquisition unit may include at least one electrode, at least one optical emitter, and at least one optical receiver.

The at least one electrode is arranged at a target part of an object, and is used to acquire an electroencephalogram signal sequence of the object. The object includes a target object or a sample object, and the electroencephalogram signal sequence includes a target electroencephalogram signal sequence or a sample electroencephalogram signal sequence.

The at least one optical emitter is arranged at the target part, and is used to emit incident light.

The at least one optical receiver is arranged at the target part, and is used to: acquire an exit optical signal formed by an exit of the incident light passing through the target part, and convert the exit optical signal into an electrical signal.

The first processor is used to obtain a blood oxygen signal sequence of the object according to the electrical signal, and the blood oxygen signal sequence includes a target blood oxygen signal sequence or a sample blood oxygen signal sequence.

According to embodiments of the present disclosure, a positional relationship between the electrode, the optical emitter and the optical receiver in the signal acquisition unit includes one of the following.

Each optical emitter corresponds to an optical receiver, and an electrode is arranged between the optical emitter and the optical receiver; each optical emitter corresponds to two optical receivers, the two optical receivers are separated from the optical emitter by a same distance, and an electrode is arranged between the optical emitter and each of the two optical receivers; an electrode is arranged between two adjacent optical emitters, and an electrode is arranged between two adjacent optical receivers; or each optical emitter corresponds to two optical receivers, the two optical receivers are separated from the optical emitter by different distances, and an electrode is arranged between the optical emitter and each of the two optical receivers.

According to embodiments of the present disclosure, the signal acquisition module may include at least one electrode, at least one optical emitter, at least one optical receiver, and a first processor. The first processor may obtain the target blood oxygen signal sequence and the sample blood oxygen signal sequence of the object according to the electrical signal converted by the optical receiver; determine the current state of the object according to the determined target blood oxygen signal sequence; and determine the sample state classification result of the object according to the determined sample blood oxygen signal sequence.

According to embodiments of the present disclosure, the electronic device may further include a second processor.

The second processor is used to turn on the optical emitter and the optical receiver when the state of the object is determined to be an expected state according to the original electroencephalogram signal sequence.

According to embodiments of the present disclosure, the signal acquisition module may further include a second processor, which may be used to turn on the optical emitter and the optical receiver when the state of the object is determined to be the expected state according to the original electroencephalogram signal sequence.

According to embodiments of the present disclosure, with the signal acquisition unit including at least one electrode, at least one optical emitter, at least one optical receiver, and a first processor, an operation mode of the electronic device may be adjusted according to the state of the object, so as to save energy consumption.

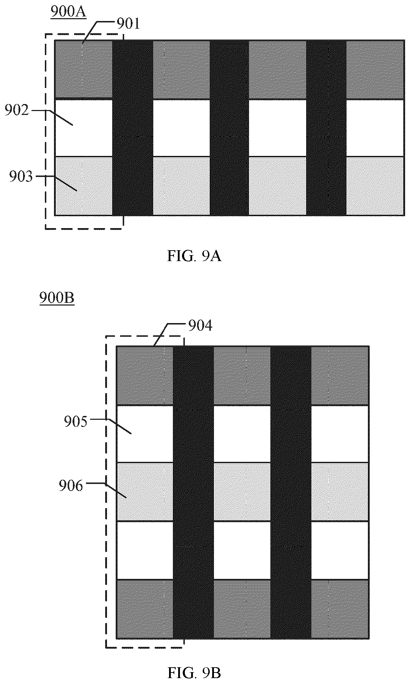

FIG. 9A schematically shows a block diagram of an electronic device suitable for implementing the method of determining the state of the object and the method of training the deep learning model according to embodiments of the present disclosure.

As shown in FIG. 9A, an electronic device 900A may include at least one optical receiver 901, at least one electrode 902, and at least one optical emitter 903. In this case, one optical receiver 901, one electrode 902 and one optical emitter 903 are used as a group, and the electrode 902 is arranged between the optical receiver 901 and the optical emitter 903 as a barrier.

FIG. 9B schematically shows a block diagram of an electronic device suitable for implementing the method of determining the state of the object and the method of training the deep learning model according to other embodiments of the present disclosure.

As shown in FIG. 9B, an electronic device 900B may include at least one optical receiver 904, at least one electrode 905, and at least one optical emitter 906. In this case, two optical receivers 904, two electrodes 905 and one optical emitter 906 are used as a group to reduce the number of optical emitters and reduce costs. In addition, two channels are formed for each sampling point, and each signal channel may provide a separate blood oxygen signal to the processor, so that detection accuracy may be improved by utilizing data from a plurality of channels.

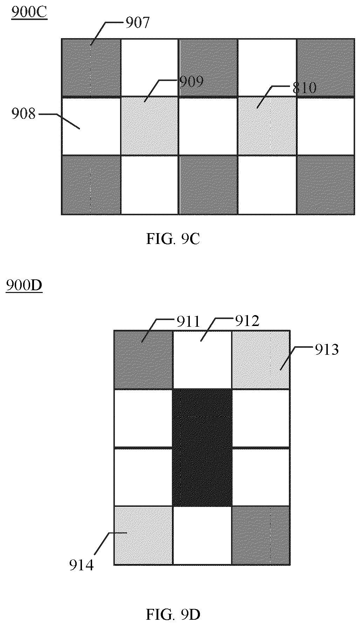

FIG. 9C schematically shows a block diagram of an electronic device suitable for implementing the method of determining the state of the object and the method of training the deep learning model according to other embodiments of the present disclosure.

As shown in FIG. 9C, an electronic device 900C may include at least one optical receiver 907, at least one electrode 908, and at least one optical emitter 909. In this case, the optical receiver 907 and the optical emitter 909 are alternately spaced relative to the electrode 908, and two adjacent optical emitters 909 may be set with different wavelengths. Different optical emitters 909 may be turned on for different operation modes to ensure an array density of the electrodes and improve a space utilization.

For example, when it is required to collect oxyHb data and deoxyHb data, an emission wavelength of the optical emitter 909 may be set to near-infrared light at 720 to 790 nm, and the optical emitter 909 may be used to collect deoxyHb data; the emission wavelength of the optical emitter 910 may be set to near-infrared light at 870 to 920 nm, and the optical emitter 910 may be used to collect oxyHb data.

FIG. 9D schematically shows a block diagram of an electronic device suitable for implementing the method of determining the state of the object and the method of training the deep learning model according to other embodiments of the present disclosure.

As shown in FIG. 9D, an electronic device 900D may include at least one optical receiver 911, at least one electrode 912, and at least one optical emitter 913. In this case, the optical emitter 913 and the optical emitter 914 may be arranged opposite the optical receiver 911, the optical emitter 913 and the optical receiver 911 may be used as a first channel, and the optical emitter 914 and the optical receiver 911 may be used as a second channel, so as to collect feature data from different spaces.

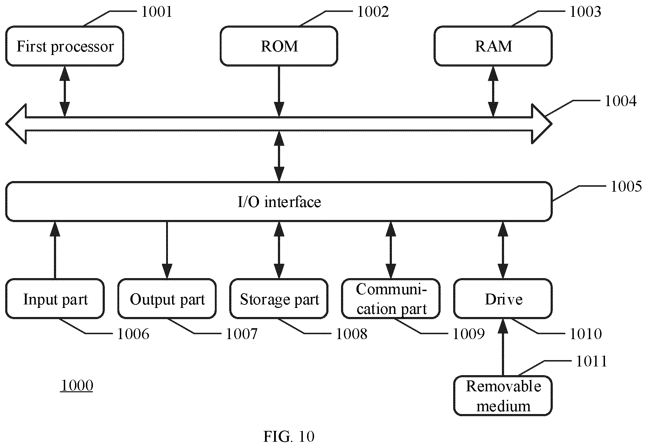

FIG. 10 schematically shows a block diagram of an electronic device suitable for implementing the method of determining the state of the object and the method of training the deep learning model according to other embodiments of the present disclosure. The electronic device shown in FIG. 10 is merely an example, and should not bring any limitation to functions and scopes of use of embodiments of the present disclosure.

As shown in FIG. 10, an electronic device 1000 according to embodiments of the present disclosure includes a first processor 1001, which may execute various appropriate actions and processing according to the program stored in a read only memory (ROM) 1002 or the program loaded into a random access memory (RAM) 1003 from a storage part 1008. The first processor 1001 may, for example, include a general-purpose microprocessor (for example, CPU), an instruction set processor and/or a related chipset and/or a special-purpose microprocessor (for example, an application specific integrated circuit (ASIC)), and the like. The first processor 1001 may further include an on-board memory for caching purposes. The first processor 1001 may include a single processing unit or plurality of processing units for executing different actions of the method flow according to embodiments of the present disclosure.

Various programs and data required for the operation of the device 1000 are stored in the RAM 1003. The processor 1001, the ROM 1002 and the RAM 1003 are connected to each other through a bus 1004. The first processor 1001 executes various operations of the method flow according to embodiments of the present disclosure by executing the programs in the ROM 1002 and/or the RAM 1003. It should be noted that the program may also be stored in one or more memories other than the ROM 1002 and the RAM 1003. The first processor 1001 may also execute various operations of the method flow according to embodiments of the present disclosure by executing the programs stored in the one or more memories.

According to embodiments of the present disclosure, the electronic device 1000 may further include an input/output (I/O) interface 1005 which is also connected to the bus 1004. The device 1000 may further include one or more of the following components connected to the I/O interface 1005: an input part 1006 including a keyboard, a mouse, etc.; an output part 1007 including a cathode ray tube (CRT), a liquid crystal display (LCD), etc. and a speaker, etc.; a storage part 1008 including a hard disk, etc.; and a communication part 1009 including a network interface card such as a LAN card, a modem, and the like. The communication part 1009 performs communication processing via a network such as the Internet. A drive 1010 is also connected to the I/O interface 1005 as required. A removable medium 1011, such as a magnetic disk, an optical disk, a magneto-optical disk, a semiconductor memory, and the like, is installed on the drive 1010 as required, so that the computer program read therefrom is installed into the storage part 1008 as needed.

The method flow according to embodiments of the present disclosure may be implemented as a computer software program. For example, embodiments of the present disclosure include a computer program product including a computer program carried on a computer-readable storage medium. The computer program includes a program code for execution of the method shown in the flowchart. In such embodiments, the computer program may be downloaded and installed from the network through the communication part 1009, and/or installed from the removable medium 1011. When the computer program is executed by the processor 1001, the above-mentioned functions defined in the system of embodiments of the present disclosure are performed. According to embodiments of the present disclosure, the above-described systems, apparatuses, devices, modules, units, etc. may be implemented by computer program modules.

The present disclosure further provides a computer-readable storage medium, which may be included in the apparatus/device/system described in the above embodiments; or exist alone without being assembled into the apparatus/device/system. The above-mentioned computer-readable storage medium carries one or more programs that when executed, perform the methods according to embodiments of the present disclosure.

According to embodiments of the present disclosure, the computer-readable storage medium may be a non-transitory computer-readable storage medium, for example, may include but not limited to: a portable computer disk, a hard disk, a random access memory (RAM), a read-only memory (ROM), an erasable programmable read-only memory (EPROM or flash memory), a portable compact disk read-only memory (CD-ROM), an optical storage device, a magnetic storage device, or any suitable combination of the above. In the present disclosure, the computer-readable storage medium may be any tangible medium that contains or stores programs that may be used by or in combination with an instruction execution system, apparatus or device.

For example, according to embodiments of the present disclosure, the computer-readable storage medium may include the above-mentioned ROM 1002 and/or RAM 1003 and/or one or more memories other than the ROM 1002 and RAM 1003.

Embodiments of the present disclosure further include a computer program product, which contains a computer program. The computer program contains program code for performing the method provided by the embodiments of the present disclosure. When the computer program product runs on an electronic device, the program code causes the electronic device to implement the method of determining the state of the object and the method of training the deep learning model provided in embodiments of the present disclosure.

When the computer program is executed by the first processor 1001, the above-mentioned functions defined in the system/apparatus of the embodiments of the present disclosure are performed. According to the embodiments of the present disclosure, the above-described systems, apparatuses, modules, units, etc. may be implemented by computer program modules.

In an embodiment, the computer program may rely on a tangible storage medium such as an optical storage device and a magnetic storage device. In another embodiment, the computer program may also be transmitted and distributed in the form of signals on a network medium, downloaded and installed through the communication part 1009, and/or installed from the removable medium 811. The program code contained in the computer program may be transmitted by any suitable medium, including but not limited to a wireless one, a wired one, or any suitable combination of the above.

According to the embodiments of the present disclosure, the program code for executing the computer programs provided by the embodiments of the present disclosure may be written in any combination of one or more programming languages. In particular, these computing programs may be implemented using high-level procedures and/or object-oriented programming languages, and/or assembly/machine languages. Programming languages include, but are not limited to, Java, C++, Python, "C" language or similar programming languages. The program code may be completely executed on the user computing device, partially executed on the user device, partially executed on the remote computing device, or completely executed on the remote computing device or server. In a case of involving a remote computing device, the remote computing device may be connected to a user computing device through any kind of network, including a local area network (LAN) or a wide area networks (WAN), or may be connected to an external computing device (e.g., through the Internet using an Internet service provider).

The flowcharts and block diagrams in the accompanying drawings illustrate the possible architecture, functions, and operations of the system, method, and computer program product according to various embodiments of the present disclosure. In this regard, each block in the flowcharts or block diagrams may represent a part of a module, a program segment, or a code, which part includes one or more executable instructions for implementing the specified logical function. It should be further noted that, in some alternative implementations, the functions noted in the blocks may also occur in a different order from that noted in the accompanying drawings. For example, two blocks shown in succession may actually be executed substantially in parallel, or they may sometimes be executed in a reverse order, depending on the functions involved. It should be further noted that each block in the block diagrams or flowcharts, and the combination of blocks in the block diagrams or flowcharts, may be implemented by a dedicated hardware-based system that performs the specified functions or operations, or may be implemented by a combination of dedicated hardware and computer instructions. Those skilled in the art may understand that the various embodiments of the present disclosure and/or the features described in the claims may be combined in various ways, even if such combinations are not explicitly described in the present disclosure. In particular, without departing from the spirit and teachings of the present disclosure, the various embodiments of the present disclosure and/or the features described in the claims may be combined in various ways. All these combinations fall within the scope of the present disclosure.

Embodiments of the present disclosure have been described above. However, these embodiments are for illustrative purposes only, and are not intended to limit the scope of the present disclosure. Although the various embodiments have been described separately above, this does not mean that measures in the respective embodiments may not be used in combination advantageously. The scope of the present disclosure is defined by the appended claims and their equivalents. Those skilled in the art may make various substitutions and modifications without departing from the scope of the present disclosure, and these substitutions and modifications should all fall within the scope of the present disclosure.

**Claims**

1. A method of determining a state of an object, comprising:

   performing a feature extraction on a target electroencephalogram signal sequence and a target blood oxygen signal sequence of a target object respectively, so as to obtain target electroencephalogram feature data and target blood oxygen feature data;
   determining a correlation between the target electroencephalogram feature data and the target blood oxygen feature data;
   performing a feature fusion on the target electroencephalogram feature data and the target blood oxygen feature data based on the correlation between the target electroencephalogram feature data and the target blood oxygen feature data, so as to obtain target fusion feature data; and
   determining a current state of the target object according to the target fusion feature data.

2. The method according to claim 1, wherein the determining a correlation between the target electroencephalogram feature data and the target blood oxygen feature data comprises:

determining a first query matrix and a first key matrix according to the target electroencephalogram feature data;
determining a second query matrix and a second key matrix according to the target blood oxygen feature data;
determining a first attention weight matrix according to the first query matrix and the second key matrix;
determining a second attention weight matrix according to the second query matrix and the first key matrix; and
determining the correlation between the target electroencephalogram feature data and the target blood oxygen feature data according to the first attention weight matrix and the second attention weight matrix.

3. The method according to claim 2, wherein the performing a feature fusion on the target electroencephalogram feature data and the target blood oxygen feature data based on the correlation between the target electroencephalogram feature data and the target blood oxygen feature data to obtain target fusion feature data comprises:

obtaining a first output feature vector according to the first attention weight matrix and a second value matrix, wherein the second value matrix is obtained according to the target blood oxygen feature data;
obtaining a second output feature vector according to the second attention weight matrix and a first value matrix, wherein the first value matrix is obtained according to the target electroencephalogram feature data; and
obtaining the target fusion feature data according to the first output feature vector and the second output feature vector.

4. The method according to any one of claims 1 to 3, further comprising:

dividing an original electroencephalogram signal sequence and an original blood oxygen signal sequence of the target object respectively, so as to obtain a plurality of original electroencephalogram signals and a plurality of original blood oxygen signals;
obtaining the target electroencephalogram signal sequence according to the plurality of original electroencephalogram signals; and
obtaining the target blood oxygen signal sequence according to the plurality of original blood oxygen signals.

5. The method according to claim 4, wherein the dividing an original electroencephalogram signal sequence and an original blood oxygen signal sequence of the target object respectively to obtain a plurality of original electroencephalogram signals and a plurality of original blood oxygen signals comprises:
dividing the original electroencephalogram signal sequence and the original blood oxygen signal sequence of the target object respectively based on a sliding window method, so as to obtain the plurality of original electroencephalogram signals and the plurality of original blood oxygen signals.

6. The method according to any one of claims 1 to 4, wherein the performing a feature extraction on a target electroencephalogram signal sequence and a target blood oxygen signal sequence of a target object respectively to obtain target electroencephalogram feature data and target blood oxygen feature data comprises:
performing a local feature extraction on the target electroencephalogram signal sequence and the target blood oxygen signal sequence of the target object respectively, so as to obtain the target electroencephalogram feature data and the target blood oxygen feature data.

7. The method according to any one of claims 1 to 4, wherein the performing a feature extraction on a target electroencephalogram signal sequence and a target blood oxygen signal sequence of a target object respectively to obtain target electroencephalogram feature data and target blood oxygen feature data comprises:
performing a global feature extraction on the target electroencephalogram signal sequence and the target blood oxygen signal sequence of the target object respectively, so as to obtain the target electroencephalogram feature data and the target blood oxygen feature data.

8. The method according to any one of claims 1 to 4, wherein the performing a feature extraction on a target electroencephalogram signal sequence and a target blood oxygen signal sequence of a target object respectively to obtain target electroencephalogram feature data and target blood oxygen feature data comprises:

performing a local feature extraction on the target electroencephalogram signal sequence and the target blood oxygen signal sequence of the target object respectively, so as to obtain intermediate target electroencephalogram feature data and intermediate target blood oxygen feature data; and

performing a global feature extraction on the intermediate target electroencephalogram feature data and the intermediate target blood oxygen feature data respectively, so as to obtain the target electroencephalogram feature data and the target blood oxygen feature data.

9. The method according to any one of claims 1 to 4, wherein the determining a current state of the target object according to the target fusion feature data comprises:

obtaining a classification result according to the target fusion feature data; and
determining the current state of the target object according to the classification result.

10. The method according to any one of claims 1 to 4, further comprising:
displaying the correlation between the target electroencephalogram signal sequence and the target blood oxygen signal sequence by using a predetermined marker, wherein the correlation between the target electroencephalogram signal sequence and the target blood oxygen signal sequence is determined according to the target electroencephalogram feature data and the target blood oxygen feature data.

11. A method of training a deep learning model, comprising:

performing a feature extraction on a sample electroencephalogram signal sequence and a sample blood oxygen signal sequence of a sample object respectively, so as to obtain sample electroencephalogram feature data and sample blood oxygen feature data;
determining a correlation between the sample electroencephalogram feature data and the sample blood oxygen feature data;
performing a feature fusion on the sample electroencephalogram feature data and the sample blood oxygen feature data based on the correlation between the sample electroencephalogram feature data and the sample blood oxygen feature data, so as to obtain sample fusion feature data;
obtaining a sample state classification result of the sample object according to the sample fusion feature data; and
training the deep learning model by using the sample state classification result and a sample state label value, so as to obtain an object state determination model.

12. The method according to claim 11, wherein the training the deep learning model by using the sample state classification result and a sample state label value to obtain an object state determination model comprises:

inputting the sample state classification result and the sample state label value into a loss function to obtain an output value; and
adjusting a model parameter of the deep learning model according to the output value until a predetermined end condition is met, so as to obtain the object state determination model.

13. The method according to claim 11 or 12, further comprising: repeatedly performing an operation until a performance test result of the object state determination model meets a predetermined performance condition, wherein the operation comprises:

testing a model performance of the object state determination model by using a validation signal sequence, so as to obtain the performance test result;
adjusting a model hyper-parameter corresponding to the object state determination model, in response to a determination that the performance test result does not meet the predetermined performance condition; and
re-training the deep learning model based on the adjusted model hyper-parameter by using the sample electroencephalogram signal sequence, the sample blood oxygen signal sequence and the sample state label value, so as to obtain a new object state determination model.

14. An electronic device, comprising:

one or more first processors;
a memory for storing one or more programs,
wherein the one or more programs are configured to, when executed by the one or more first processors, cause the one or more first processors to implement the method of any one of claims 1 to 13.

15. The electronic device according to claim 14, further comprising:

a signal acquisition module comprising at least one signal acquisition unit, wherein the signal acquisition unit comprises:

at least one electrode arranged at a target part of an object, wherein the at least one electrode is configured to acquire an electroencephalogram signal sequence of the object, the object comprises a target object or a sample object, and the electroencephalogram signal sequence comprises a target electroencephalogram signal sequence or a sample electroencephalogram signal sequence;

at least one optical emitter arranged at the target part, wherein the at least one optical emitter is configured to emit incident light; and

at least one optical receiver arranged at the target part, wherein the at least one optical receiver is configured to acquire an exit optical signal formed by an exit of the incident light passing through the target part, and convert the exit optical signal into an electrical signal; and

wherein the first processor is configured to obtain a blood oxygen signal sequence of the object according to the electrical signal, and the blood oxygen signal sequence comprises a target blood oxygen signal sequence or a sample blood oxygen signal sequence.

16. The electronic device according to claim 15, wherein a positional relationship between the electrode, the optical emitter and the optical receiver in the signal acquisition unit comprises:

each optical emitter corresponds to an optical receiver, and an electrode is arranged between the optical emitter and the optical receiver;

each optical emitter corresponds to two optical receivers, the two optical receivers are separated from the optical emitter by a same distance, and an electrode is arranged between the optical emitter and each of the two optical receivers;

an electrode is arranged between two adjacent optical emitters, and an electrode is arranged between two adjacent optical receivers; or

each optical emitter corresponds to two optical receivers, the two optical receivers are separated from the optical emitter by different distances, and an electrode is arranged between the optical emitter and each of the two optical receivers.

17. The electronic device according to any one of claims 14 to 16, further comprising:
a second processor configured to turn on the optical emitter and the optical receiver in response to determining that a state of the object is an expected state according to an original electroencephalogram signal sequence.

18. A computer readable storage medium having executable instructions therein, wherein the instructions are configured to, when executed by a processor, cause the processor to implement the method of any one of claims 1 to 13.

19. A computer program product containing computer executable instructions, wherein the computer executable instructions are configured to, when executed, implement the method of any one of claims 1 to 13.

FIG. 1

<u>200</u>

S210

A feature extraction is performed on a target electroencephalogram signal sequence and a target blood oxygen signal sequence of a target object respectively, so as to obtain target electroencephalogram feature data and target blood oxygen feature data

S220

A correlation between the target electroencephalogram feature data and the target blood oxygen feature data is determined

S230

A feature fusion is performed on the target electroencephalogram feature data and the target blood oxygen feature data respectively based on the correlation between the target electroencephalogram feature data and the target blood oxygen feature data, so as to obtain target fusion feature data

S240

A current state of the target object is determined according to the target fusion feature data

FIG. 2

300A

FIG. 3A

FIG. 3B

FIG. 4

500

S510

A feature extraction is performed on a sample electroencephalogram signal sequence and a sample blood oxygen signal sequence of a sample object respectively, so as to obtain sample electroencephalogram feature data and sample blood oxygen feature data

S520

A correlation between the sample electroencephalogram feature data and the sample blood oxygen feature data is determined

S530

A feature fusion is performed on the sample electroencephalogram feature data and the sample blood oxygen feature data based on the correlation between the sample electroencephalogram feature data and the sample blood oxygen feature data, so as to obtain sample fusion feature data

S540

A sample state classification result of the sample object is obtained according to the sample fusion feature data

S550

The deep learning model is trained using the sample state classification result and a sample state label value, so as to obtain an object state determination model

FIG. 5

FIG. 6

EP 4 557 174 A1

First feature extraction module — 710

First determination module — 720

First feature fusion module — 730

Wecond determination module — 740

— 700

FIG. 7

Second feature extraction module — 810

Third determination module — 820

Second feature fusion module — 830

First obtaining module — 840

First training module 850 — 850

— 800

FIG. 8

900A

901

902

903

FIG. 9A

900B

904

905

906

FIG. 9B

900C

FIG. 9C

900D

FIG. 9D

FIG. 10

| INTERNATIONAL SEARCH REPORT | International application No. |
| --- | --- |
| | **PCT/CN2022/105583** |

**A. CLASSIFICATION OF SUBJECT MATTER**

G06N 3/08(2006.01)i

According to International Patent Classification (IPC) or to both national classification and IPC

**B. FIELDS SEARCHED**

Minimum documentation searched (classification system followed by classification symbols)

G06N, G16H, A61B

Documentation searched other than minimum documentation to the extent that such documents are included in the fields searched

Electronic data base consulted during the international search (name of data base and, where practicable, search terms used)

VEN, CNABS, CNTXT, CNKI, IEEE: 脑电波, 血氧, 融合, 深度学习, 神经网络, 相关性, 注意力, 键值, brain waves, oxygen, fusion, deep learning, neural network, correlation, attention, key value

**C. DOCUMENTS CONSIDERED TO BE RELEVANT**

| Category* | Citation of document, with indication, where appropriate, of the relevant passages | Relevant to claim No. |
| --- | --- | --- |
| Y | CN 113951900 A (YANSHAN UNIVERSITY) 21 January 2022 (2022-01-21) claims 1-10 | 1-19 |
| Y | CN 114550907 A (ZHEJIANG UNIVERSITY MEDICAL COLLEGE AFFILIATED CHILDREN HOSPITAL et al.) 27 May 2022 (2022-05-27) description, paragraphs [0052]-[0152] | 1-19 |
| A | CN 114548399 A (HANGZHOU SENSOMICS TECHNOLOGY CO., LTD.) 27 May 2022 (2022-05-27) entire document | 1-19 |
| A | US 2020086127 A1 (NEUROSTEER LTD.) 19 March 2020 (2020-03-19) entire document | 1-19 |
| A | WO 2022074300 A1 (DATAHAMMER OY) 14 April 2022 (2022-04-14) entire document | 1-19 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 December 2022** | **29 December 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

International application No.

**PCT/CN2022/105583**

| Patent document cited in search report | | | Publication date (day/month/year) | Patent family member(s) | | | Publication date (day/month/year) |
|---|---|---|---|---|---|---|---|
| CN | 113951900 | A | 21 January 2022 | None | | | |
| CN | 114550907 | A | 27 May 2022 | None | | | |
| CN | 114548399 | A | 27 May 2022 | None | | | |
| US | 2020086127 | A1 | 19 March 2020 | CA | 3113322 | A1 | 26 March 2020 |
| | | | | US | 2021023378 | A1 | 28 January 2021 |
| | | | | WO | 2020058761 | A1 | 26 March 2020 |
| | | | | EP | 3876834 | A1 | 15 September 2021 |
| WO | 2022074300 | A1 | 14 April 2022 | EP | 4021288 | A1 | 06 July 2022 |
| | | | | FI | 20205993 | A1 | 10 April 2022 |

Form PCT/ISA/210 (patent family annex) (January 2015)